Europäisches Patentamt

**European Patent Office**

Office européen des brevets

(11) Publication number: **0 058 575**
**A1**

(12) # EUROPEAN PATENT APPLICATION

(21) Application number: **82300814.9**

(22) Date of filing: **17.02.82**

(51) Int. Cl.³: **A 61 K 35/74**
**A 61 K 7/16**

(30) Priority: **17.02.81 US 235130**

(43) Date of publication of application:
**25.08.82 Bulletin 82/34**

(84) Designated Contracting States:
**AT CH DE FR GB LI NL SE**

(71) Applicant: **FORSYTH DENTAL INFIRMARY FOR CHILDREN**
**140 Fenway**
**Boston Massachusetts 02115(US)**

(72) Inventor: **Hillman, Jeffrey D.**
**162 Hillside Street Roxbury Crossing**
**Boston Massachusetts(US)**

(74) Representative: **Myerscough, Philip Boyd et al,**
**J.A.Kemp & Co. 14, South Square Gray's Inn**
**London, WC1R 5EU(GB)**

(54) Microorganisms for use in treating oral cavity diseases.

(57) *Streptococcus santuis*, *Streptococcus uberis* or *Actinomyces bovis* are useful for the treatment of diseases of the oral cavity, particular periodontal diseases such as periodontosis and periodontitis. These microorganisms inhibit the growth of *Actinobacillus actinomycetemcomitans* which causes such diseases. The microorganisms can be used in the form of a pharmaceutical composition, such as a mouthwash also incorporating suitable carriers or diluents.

EP 0 058 575 A1

DESCRIPTION

TITLE: MICROORGANISMS FOR USE IN TREATING ORAL CAVITY DISEASES

This invention relates to a microorganism or microorganisms for use in the treatment of a disease of the oral cavity, a pharmaceutical composition suitable for use in such treatment and a method of inhibiting the growth of the microorganism Actinobacillus actinomycetemcomitans.

Recent studies have implicated Actinobacillus actinomycetemcomitans as a possible etiologic agent of juvenile periodontitis (periodontosis). The organism has been recovered with greater frequency and in higher numbers from lesions of periodontosis than healthy sites in the same individuals or from lesions of gingivitis or periodontitis (Mandell, R.L. and Socransky, S.S. (1980), A selective medium for Actinobacillus actinomycetemcomitans. J. Dent. Res. (Special Issue A, Abstract 972); and Slots, J., Reynolds, H.S., Lobbins, P.N. and Genco R.J. (1980), Actinobacillus actinomycetemcomitans: Selective culturing and oral ecology in patients with localized juvenile periodontitis. J. Dent. Res. 59: (Special Issue A, Abstract 244)). Serum antibody titers to the organism are more frequently elevated in patients with periodontosis, when compared to healthy individual and individuals with other forms of periodontal disease (Ebersole,J.L., Frey, D.E., Taubman, M.A., Smith, D.J. and Genco, R.J. (1980), Serum antibody response to A. actinomycetemcomitans (Y4) in periodontal diseases, J. Dent. Res. 59: (Special Issue A, Abstract 249); Ebersole, J.L., Frey, D.E., Taubman, M.A., Smith, D.J., Genco, R.J. and Hammond, B.F. (1980), Antibody response to antigens from A. actinomycetemcomitans (Y4): Relationship to localized juvenile periodontitis (LJP). J. Dent. Res. 59: (Special Issue A, Abstract 255); Genco, R.J., Taichman, N.A. and Sadowski, C.A. (1980), Precipitating antibodies to Actinobacillus actinomycetemcomitans in localized juvenile periodontitis. J. Dent. Res 59: (Special Issue A, Abstract 246); and Lai, C.H. and Listgarten, M.A. (1980), Circulating antibody titers to Actinobacillus actinomycetemcomitans in patients with periodontal disease. J. Dent. Res. 59: (Special Issue A, Abstract 957)).

Strains of Actinobacillus actinomycetemcomitans produce a toxin capable of lysing polymorphonuclear leukocytes (Baehni, P., Tsai, C., McArthur, W.P., Hammond B.F. and Taichman, N.S. (1979), VII. Dectection of leukotoxic activity of a plaque derived gram negative organism. Infect. and Immun. 24: 233-243; and Baehni, P., Tsai, C., McArthur, W.P., Hammond B.F., Socransky, S.S. and Taichman, N.S. (1980), Leukotoxicity of various Actinobacilli actinomycetemcomitans. J. Dent. Res. 59: (Special Issue A, Abstract 223)). Antibodies to this toxin may be detected in periodontosis patients which are protective against the toxins lytic effect(McArthur, W.P., Tsai, C.C., Baehni, P., Genco, R.J. and Taichman, N. (1980), Modulation of Actinobacillus actinomycetemcomitans (Y4) leukotoxicity by serum. J. Dent. Res. 59: (Special Issue A, Abstract 225)). Actinobacillus actinomycetemcomitans has been shown to accelerate alveolar bone loss in gnotobiotic rats: Irving, J.T., Newman, M.G., Socransky, S.S. and Heely, J.D. (1975), Histological changes in experimental periodontal disease in rats monoinfected with a gram negative organism. Arch. Oral Biol. 20: 219-220. Actinobacillus actinomycetemcomitans does not appear to be a numerically dominant member of the microbiota of individuals without adolescent destructive disease, but can attain proportions as high as 70% of the cultivable microbiota in sites of advanced destruction.

According to the present invention, there is provided a microorganism or microorganisms for use in the treatment of a disease of the oral cavity, the or each microorganism being a strain of <u>Streptococcus sanguis</u>, <u>Streptococcus uberis</u> or <u>Actinomyces bovis</u>.

The present invention further provides a pharmaceutical composition suitable for use in the treatment of a disease of the oral cavity, which comprises at least one strain of microorganism selected from <u>Streptococcus sanguis</u>, <u>Streptococcus uberis</u> and <u>Actinomyces bovis</u>, together with a suitable carrier or diluent.

The invention also provides a method of inhibiting the growth of the microorgansim <u>Actinobacillus actinomycetemcomitans</u>, which method comprises contacting the <u>Actinobacillus actinomycetemcomitans</u> with an effective inhibitory amount of an effector strain selected from <u>Streptococcus sanguis</u>, <u>Streptococcus uberis</u> and <u>Actinomyces bovis</u>, and combinations thereof, with the proviso that said method is not a method of treatment of the human or animal body by surgery or therapy or a diagnostic method practised on the human or animal body.

It has been discovered that certain microorganisms resident in healthy periodontal sites, or sites with a different form of the periodontal disease, prevent the establishment or over-growth of <u>Actinobacillus actinomycetemcomitans</u>. It has been found that the growth of <u>Actinobacillus actinomycetemcomitans</u> is inhibited by the application of <u>Streptococcus sanguis</u>, <u>Streptococcus uberis</u> and <u>Actinomyces bovis</u>, alone or in combination. <u>Streptococcus uberis</u> and, particularly, <u>Streptococcus sanguis</u> are preferred. The microorganisms and pharmaceutical compositions containing them can therefore be used in the treatment of diseases of the oral cavity, particularly periodontal diseases such as periodontosis and periodontitis, more particularly for the treatment and prevention of juvenile periodontosis characterized by a localized pattern of destruction and familial tendency. The invention provides a means for the bacterial interference with the growth of Actinobacillus actinomycetemcomitans.

The application of the effector strain of Streptococcus sanguis, Streptococcus uberis and Actinomyces bovis to the oral cavity of a patient, and particularly when placed in direct contact with affected tooth or teeth surfaces, prevents and cures periodontosis. The effector strain microorganism discovered may be introduced into the oral cavity in a number of forms and compositions, such as by the use of mouthwashes, mouthrinses, spray solutions and swabbing solutions or suspensions, by swabbing, spraying, rinsing, etc., techniques. In one method, a cell suspension of the selected effector strains, or combinations thereof, is swabbed directly onto and about the affected tooth or teeth surface, or the area to be protected, one or more times over a selected period of treatment time, depending on the patient and extent of the disease, until the periodontal disease is cured or prevented. A typical swabbing solution or suspension would include a culture medium or broth containing an effective or inhibitory amount of the effector strain therein. A swabbing solution would comprise, for example, a commercial Todd-Hewitt broth supplemented with a carbohydrate or sugar, such as a small amount of glucose of 0.1% to 2.0%, for example, .50%, with about $10^9$ colonies/ml or more of the effector strain microorganism.

Microorganisms for use in the present invention have been isolated and characterized and found to be the same or similar to known microorganisms identified as Streptococcus uberis, Streptococcus sanguis and Antinomyces bovis. For example, strains identified herein as KJ2 (Steptococcus uberis) and KJ3 (Streptococcus sanguis) have been isolated, characterized and discovered to be effective in preventing the establishment or growth of Actinobacillus actinomycetemcomitans. Strain KJ2 has been found to be the same as Streptococcus uberis strain ATCC 10557 and strain KJ2 has been found to be the same as Streptococcus sanguis type II strain ATCC 19436. Strains KJ2 and KJ3 were isolated as follws:

Subgingival plaque was obtained from the first molar region of a clinically normal adult female. The sample was diluted in sterile phosphate-buffered saline (pH 7.0) and spread on blood agar plates, to give isolated colonies. Following three days incubation at 37°C in an atmosphere of 80% $N_2$, 10% $H_2$ and 10% $CO_2$, single colonies which appeared were picked with sterile toothpicks and replica-stabbed into blood agar plates with and without a lawn of Actinobacillus actinomycetemcomitans strain Y4. After two days incubation at 37°C in an atmosphere of 80% $N_2$, 10% $H_2$ and 10% $CO_2$, the stabs were examined for zones of inhibition of the Actinobacillus lawn. KJ2 and KJ3 were isolated from the replica of stabs, which produced clear zones of inhibition of the Actinobacillus lawn measuring ca. 3mm.

As far as the characterization of strains KJ2 and KJ3 is concerned, both produce small α hemolytic colony types on trypticase soy agar containing 5% sheep red blood cells. Both strains are gram-positive cocci, typically presented in pairs and chains.

Strain KJ2 has been identified as a representative of the class of bacteria called Streptococcus uberis on basis of the following tests: gas liquid chromatography of culture liquors indicate lactic, formic and acetic acids to be the chief end-products of glucose fermentation. It can ferment the following compounds to produce acid: arabinose, cellobiose, glucose, glycerol, inulin, lactoase, maltose, mannitol, melibiose, raffinose, salicin, sucrose, trehalose and xylose. It is negative in tests for esculin hydrolysis, nitrite and nitrate reduction, ammonia production from urea, and growth in the presence of 40% bile. The organism was found to produce ammonia from argenine and to degrade hippuric acid. It also produces hydrogen perioxide, when grown in an atmosphere of air plus 10% $CO_2$. The strain of Streptococcus uberis deposited at the ATCC under number ATCC 19436 possesses these properties.

Strain KJ3 has been identified as a representative of the class of bacteria called Streptococcus sanguis II on the basis of the following tests: gas liquid chromatography of culture liquors indicate lactic, formic and acetic acids to be the chief end-products of glucose fermentation. It can ferment the following compounds: glucose, lactose, maltose, mannitol, melibiose, raffinose, salicin, sucrose and trehalose. It cannot ferment cellobiose, glycerol, inulin, or xylose. It cannot hydrolyze esculin, reduce nitrite or nitrate, produce ammonia from argenine or urea, degrade hippuric acid, or grow in the presence of 40% bile. It does produce hydrogen peroxide, when grown in an atmosphere of air plus 10% $CO_2$. The strain of Streptococcus sanguis type II deposited at the ATCC under number ATCC 10557 possesses these properties.

The following Examples illustrate the invention Example 1

Bacterial Strains and Media. Cultures of Actinobacillus actinomycetemcomitans strain Y4 were grown in mycoplasma broth (Baltimore Biologic Laboratories, Cockeysville, Maryland), supplemented with 0.1% glucose, 5 µg/ml hemin, and 1 mg/ml sodium bicarbonate as previously described. The medium was inoculated from 50% (v/v) glycerol stabs maintained at -20°C and incubated aerobically overnight at 37°C.

Plaque Sampling. Supra or subgingival plaque samples were obtained from healthy and periodontosis patients, using the methods of Newman and Socransky (Newman, M.G.,

Socransky, S.S. (1977), Predominant cultivable microbiota in periodontosis. J. Perio. Res. 12: 120-128). The samples were dispersed by sonication for 10 seconds with an MSE sonic oscillator and serial 10-fold diluted samples spread on trypticase soy agar (BBL) plates, supplemented with 5% sheep blood. The plates were incubated for 3 to 4 days at 37°C in an atmosphere of 80% $N_2$, 10% $CO_2$ and 10% $H_2$.

Inhibitor Screening. A lawn of strain Y4 was prepared on chocolate agar medium by cross-streaking 0.2 ml of an overnight culture diluted 1:1000 with phosphate-buffered saline (pH 7). One hundred to five hundred random isolated colonies obtained from the plaque samples on blood agar plates were replica-stabbed, using sterile toothpicks, onto chocolate agar plates with and without a lawn of strain Y4. The plates were then incubated for 2 days at 37°C in an atmosphere of 80% $N_2$, 10% $CO_2$ and 10% $H_2$. Interference was considered present, when direct visual examination revealed a zone of complete inhibition surrounding the stab. Results are expressed as the percentage of plaque isolates tested which inhibited the growth of strain Y4.

Results

Plaque samples obtained from seven subjects, who were free of clinically evident periodontal pathology, were analyzed for their content of inhibitory organisms to Actinobacillus actinomycetemcomitans. As seen in Table 1, it was found that all but one of the eleven sites samples harbored such organisms.

By contrast, fifteen or sixteen plaque samples, obtained from active disease sites of periodontosis patients, failed to demonstrate the presence of inhibitors. The samples were taken from a total of sixteen supra and subgingival sites representing six different patients. Five of these subjects demonstrated clinical manifestations of uncomplicated juvenile periodontosis. The sixth subject demonstrated a characteristic Papillon LeFevre syndrome. Interestingly, four of the five healthy sites, which were samples from three of these patients, demonstrated the presence of inhibitory strains and in proportions comparable to plaque samples from nondiseased subjects.

Plaque samples from the four first molars of the mother and three siblings of one periodontosis subject were analyzed for their content of inhibitory organisms. As seen in Table 2, plaque from the mother, who has a history of destructive periodontal disease beginning in adolescence and currently presents with advanced generalized destructive disease, lacked inhibitory organisms in all of the four sites sampled. All of the siblings, aged 7, 9 and 11, lacked inhibitors in at least one of the four sites tested, and, in the case of one subject, all four of the sites were found to be affected.

## TABLE 1

Percentage of Isolates Inhibitory to
<u>Actinobacillus actinomycetemcomitans</u>

| Sites | Periodontosis Patients | | Healthy Subjects |
| | Diseased | Healthy | Healthy |
|---|---|---|---|
| | 0 | | |
| | 0 | | |
| | 0 | | |
| | 0 | 0 | 0 |
| | 0 | 5.0 | 1.6 |
| | 0 | 8.4 | 2.8 |
| | 0 | 16.4 | 4.3 |
| | 0 | 44.0 | 5.3 |
| | 0 | | 5.8 |
| | 0 | | 7.0 |
| | 0 | | 9.0 |
| | 0 | | 13.0 |
| | 0 | | 15.8 |
| | 0 | | 29.0 |
| | 0 | | |
| | $\frac{32}{0}$ | | |
| Median | 0 | 8.4 | 5.8 |
| 95% Confidence Interval to Median | 0-0 | 0-44* | 1.6-15.8 |

*93.75% confidence interval of the median, since only 5
samples studied.

Differences between groups significant at $p < 0.01$ using
Kruskal-Wallis test.

## TABLE 2

Percentage of Isolates Inhibitory to
<u>Actinobacillus actinomycetemcomitans</u>
in Plaque from Mother and Siblings of
Periodontosis Patient

| Subject | Inhibitors (%) |
|---------|----------------|
| Mother | 0 |
|  | 0 |
|  | 0 |
|  | 0 |
| Sibling | 23 |
|  | 1 |
|  | 0 |
|  | 5 |
| Sibling | 0 |
|  | 0 |
|  | 0 |
|  | 0 |
| Sibling | 42 |
|  | 9 |
|  | 7 |
|  | 0 |

Example 2

The potential usefulness of strains KJ2 and KJ3 as effector strains, in the prevention and/or treatment of juvenile periodontosis, relates to their ability to inhibit the growth of <u>Actinobacillus actinomycetemcomitans</u>, the etiologic agent of this disease.

Three groups of five germ-free Sprague-Dawley rats were maintained in separate stainless-steel isolators. At 21 days of age, animals in isolator 1 were infected by swabbing their oral cavities with an overnight culture of KJ2. Animals in isolator 2 were similarly treated with a culture of KJ3. Animals in isolator 2 were sham-infected with sterile medium. Two weeks later, animals in all three isolators were challenged by swabbing the oral cavities with a culture of Y4 diluted to give ca. $10^4$ c.f.u./ ml. At weekly intervals, plaque and saliva samples were obtained, and then the animals were reinfected, using a 10-fold higher concentration of Y4 than that used the previous week. It was found that animals in isolator 3 (sham-infected) became uniformly infected with Y4, when challenged with a concentration of cells equal to $10^6$ c.f.u./ml. By contrast, animals previously infected with KJ2 (isolator 1) or KJ3 (isolator 2) were not infected by Y4, even when challenged with concentrations of cells as high as $10^9$ c.f.u./ml. Thus, by preventing the colonization of the oral cavity by <u>Actinobacillus actinomycetemcomitans</u>, it is presumed that establishment of KJ2 and KJ3 in the mounths of individuals at risk could provide lifelong protection from juvenile periodontosis.

In a second experiment, two groups of two germ-free rats were monoinfected at 21 days of age with <u>Actinobacillus actinomycetemcomitans</u> strain Y4 by swabbing their oral cavity with an overnight culture. During the next four weeks, it was determined that Y4 had successfully colonized the oral cavities of these animals. One group of rats was then challenged with KJ2 or KJ3. At weekly intervals thereafter, the oral cavities of these animals were sampled, to determine the presence of Y4 and KJ2 and KJ3. It was found that animals challenged with KJ2 maintained their infection with Y4 although they did become colonized by KJ2. By contrast, animals challenged with KJ3 were free of Y4 infection and were heavily colonized by KJ3 after one week. These results indicate the potential usefulness of KJ3 as an agent for the treatment of active juvenile periodntosis.

The role of Actinobacillus actinomycetemcomitans in adolescent, destructive, periodontal diseases remains to be established indisputably. However, it appears that healthy individuals and individuals with other forms of periodontal disease harbor organisms, as discovered, in the gingival crevice area, which are inhibitory to Actinobacillus actinomycetemcomitans or other putative pathogens, making it difficult for the pathogen to establish or achieve sufficient numbers to initiate or maintain local pathology. Whether Actinobacillus actinomycetemcomitans is the pathogen in the affected adolescents or not, it seems clear that its presence in the lesion was not inhibited by associated microorganisms. It has been established that the absence of low numbers in individuals of Actinobacillus actinomycetemcomitans, without adolescent, destructive, periodontal disease, may be attributed at least in part to the presence of the discovered strains of antagonistic microorganisms. The mother and siblings of one periodontosis patient had one or more sites which lacked inhibitors. In the case of the siblings, no overt periodontal pathology was evident. The mechanism of inhibition by the discovered inhibitory organisms is not known. It does not appear to be acid formation, since carbohydrate in the medium was limited, and pure cultures of highly acidogenic organisms, such as Streptococcus mutans, were not inhibitory. Example 2 established that the microorganisms KJ2 and KJ3 prevented periodontosis (experiment 1), and KJ3 cured periodontosis (experiment 2), although other experiments have also shwon that KJ2 also partially cures, and that the Actinomyces bovis also is an effective, but not preferred, microorganism.

CLAIMS

1. A microorganism or microorganisms for use in the treatment of a disease of the oral cavity, the or each microorganism being a strain of Streptococcus sanguis, Streptococcus uberis or Actinomyces bovis.

2. A microorganism or microorganisms according to claim 1 for use in the treatment of a periodontal disease.

3. A microorganism or microorganisms according to claim 2 for use in the treatment of periodontosis or periodontitis.

4. A microorganism or microorganisms according to any one of the preceding claims. the microorganism(s) having the same properties as Streptococcus sanguis type II strain ATCC 10557 or Streptococcus uberis strain ATCC 19436.

5. A pharmaceutical composition suitable for use in the treatment of a disease of the oral cavity, which comprises at least one strain of microorganism selected from Streptococcus sanguis, Streptococcus uberis and Actinomyces bovis, together with a suitable carrier or diluent.

6. A composition according to claim 5 which is in the form of a mouthwash, mouthrinse, spray solution, or swabbing solution or suspension.

7. A composition according to claim 5 or 6 for use in the treatment of a disease of the oral cavity.

8.   A composition according to claim 7 for use in the treatment of a periodontal disease.

9.   A composition according to claim 8 for use in the treatment of periodontosis or periodontitis.

10.   A composition according to any one of claims 5 to 9 wherein the microorganisms(s) have the same properties as Streptococcus sanguis type II strain ATCC 10557 or Streptococcus uberis strain ATCC 19436.

11. A method  of inhibiting the growth of the microorganism Actinobacillus actinomycetemcomitans, which method comprises contacting the Actinobacillus actinomycetemcomitans with an effective inhibitory amount of an effector strain selected from Streptococcus sanguis, Streptococcus uberis and Actinomyces bovis, and combinations thereof, with the proviso that said method is not a method of treatment of the human or animal body by surgery or therapy or a diagnositic method practised on the human or animal body.

0058575

European Patent
Office

EUROPEAN SEARCH REPORT

Application number

EP 82 30 0814

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US-A-4 133 875 (HILLMAN J.D.) *The whole document and particularly page 1, column 2, lines 64-68 and page 2, column 3, lines 1-14* | 5-9 | A 61 K 35/74 A 61 K 7/16 |
| A | GB-A- 465 370 (ORA SMITH FOWLER) | | |
| A | BIOLOGICAL ABSTRACTS, vol. 62, no. 6, June 1976, pages 1363-1368, no. 32258, Philadelphia, Penncylvania (USA); J.J.BAKER et al.: "Importance of actinomyces and certain gram-negative anerobic organisms in the transformation of lymphocytes from patients with periodontal disease". & INFECT IMMUN 13(5): 1363-1368. 1976. | | |
| A | BIOLOGICAL ABSTRACTS, vol. 69, no. 7, July 1980, abstract no. 44979, Philadelphia, Pennsylvania, USA A.C.R.TANNER et al.: "Bacteria associated with advancing periodontitis in man". & J. CLIN PERIODONTOL 6(5): 278-307. 1979. | | |

TECHNICAL FIELDS SEARCHED (Int. Cl. ³)

A 61 K

The present search report has been drawn up for all claims

| Place of search | Date of completion of the search | Examiner |
|---|---|---|
| THE HAGUE | 27-05-1982 | MARIE A.L.L. |

**European Patent Office**

**EUROPEAN SEARCH REPORT**

| DOCUMENTS CONSIDERED TO BE RELEVANT | | | Page 2 |
|---|---|---|---|
| Category | Citation of document with indication, where appropriate, of relevant passages | Relevant to claim | CLASSIFICATION OF THE APPLICATION (Int. Cl. 3) |
| P,X | JOURNAL OF DENTAL RESEARCH, vol. 60, 1981, Issue A, Page 603, no. 1174; J.D.HILLMAN et al.: "Bacterial interference of actinobacillus". *The whole document* | 1-11 | |
| | ----- | | |
| | | | **TECHNICAL FIELDS SEARCHED (Int. Cl. 3)** |

The present search report has been drawn up for all claims

| Place of search | Date of completion of the search | Examiner |
|---|---|---|
| THE HAGUE | 27-05-1982 | MARIE A.L.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                         

& : member of the same patent family, corresponding document

EPO Form 1503. 03.82